(19) Europäisches Patentamt
European Patent Office
Office européen des brevets

(11) Publication number: **0 004 456**
**B1**

# (12) EUROPEAN PATENT SPECIFICATION

(45) Date of publication of patent specification: **07.04.82**

(21) Application number: **79300448.2**

(22) Date of filing: **21.03.79**

(51) Int. Cl.³: **C 07 C 9/04**, C 07 C 1/02, C 10 K 3/04

## (54) Methanation of carbon monoxide without prior separation of inert gases.

(30) Priority: **23.03.78 US 889558**

(43) Date of publication of application:
**03.10.79 Bulletin 79/20**

(45) Publication of the grant of the patent:
**07.04.82 Bulletin 82/14**

(84) Designated Contracting States:
**BE DE FR GB IT SE**

(56) References cited:
**US - A - 2 544 574**
**US - A - 3 511 624**

(73) Proprietor: **UNION CARBIDE CORPORATION**
**270, Park Avenue**
**New York, N.Y. 10017 (US)**

(72) Inventor: **Rabo, Jule Anthony**
**19 Windmill Road**
**Armonk (US)**
Inventor: **Elek, Louis Frank**
**1088 Oregon Road**
**Peekskill New York (US)**
Inventor: **Francis, James Nelson**
**7403 Santa Fe**
**Houston Texas (US)**

(74) Representative: **Blake, John Henry Francis**
**BROOKES AND MARTIN High Holborn House**
**52/54 High Holborn**
**London WC1V 6SE (GB)**

**EP 0 004 456 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Methanation of carbon monoxide without prior separation of inert gases

The invention relates to the production of methane from carbon monoxide. More particularly, it relates to a methanation process capable of effectively utilizing dilute carbon monoxide-containing gas streams.

The catalytic hydrogenation of carbon monoxide to form methane is one of the most well known and established hydrogenation reactions. This reaction, which is:

$$(1) \qquad CO+3H_2 \rightarrow CH_4+H_2O,$$

utilizes a synthesis gas, as from the gasification of coal with oxygen and steam, that is treated to provide a desired $H_2/CO$ ratio and to remove excess $CO_2$ and deleterious impurities such as sulfur compounds. As the $H_2/CO$ ratio of the raw synthesis gas is substantially below the necessary minimum ratio of 3/1, at least a portion of the carbon monoxide in the gas is generally first reacted with steam, over an iron or other suitable catalyst in the well-known "water shift" reaction, as follows:

$$(2) \qquad CO+H_2O \rightarrow CO_2+H_2.$$

Excess $CO_2$ in the gas stream is removed by conventional means, such as by treatment with alkaline absorbents. Sulfur impurities are also removed to substantially under 5 ppm, e.g., to less than about 1 ppm, preferably to less than 0.2 ppm, to protect the methanation catalyst from poisoning by such sulfur impurities.

The water shift reaction normally does not go to completion, with the equilibrium determined by the reaction temperature and other operating conditions limiting the degree of completeness of the reaction. The desired $H_2/CO$ ratio is obtained, to achieve maximum utilization of the available CO and hydrogen, either by very careful choice and control of the processing conditions or by the treatment of a portion of the raw synthesis gas to produce a $H_2/CO$ ratio substantially in excess of 3/1 and blending the treated gas with the untreated portion to produce the desired $H_2/CO$ ratio.

One variation of the latter approach is disclosed in U.S. Patent No. 3,854,895, in which the primary gas from coal gasification is divided into two streams, one of which is subjected to water shift and subsequent methanation stages, the untreated stream being added to said treated gas successively during said methanation stages. Numerous prior art techniques also exist, it should be noted, for the production of methane from other gases containing hydrogen and carbon oxides. U.S. Patent No. 3,511,624, for example, relates to the two-stage production of a gas containing a high proportion of methane from a reaction mixture comprising hydrogen, carbon monoxide and dioxide, steam and at least 25% by volume methane.

Despite the established nature of the major steps in the known techniques for the gasification of coal and in the methanation of the resulting synthesis gas, improved processes for the production of methane are urgently needed to enhance the overall economics of methane production and/or to enable its production from carbon-monoxide-containing gas streams that cannot presently be used in a commercially feasible manner for the production of methane. This need is highlighted by the diminishing supply of natural gas and the recognized need to develop economical supplies of synthetic natural gas to meet existing and anticipated requirements for low-cost, high BTU gaseous heating fuels.

In the processing of synthesis gas obtained by the gasification of coal with oxygen and steam, pretreatment by the catalytic water shift reaction is a major processing step prior to methanation. This necessary adjustment of the $H_2/CO$ ratio adds, of course, to the overall processing costs and necessarily reduces the amount of carbon monoxide available for conversion to methane. In addition, gas streams containing a low concentration of carbon monoxide and/or a high concentration of inert gases are generally not suitable for methanation purposes because of the costs associated with the concentration of the carbon monoxide, as by cryogenic or absorption means. For example, the effluent from the underground gasification of coal with air is not a suitable feed gas for conventional methanation techniques because of the high proportion of inert gases, i.e., nitrogen, in said effluent. Similarly, the effluent from blast furnace operations contains a high proportion of nitrogen and is not suitable for the economic production of methane because of the prohibitive cost of concentrating the carbon monoxide content thereof.

Conventional processing techniques in addition are known to have particular operating difficulties, the overcoming of which tends to shift the equilibrium and reduce the yield of desired methane product or tends to reduce the overall efficiency of the production reaction. Thus U.S. Patent No. 3,854,895, referred to above discloses that the formation of free carbon by the Boudouard reaction is promoted by an increase of CO in the reaction mixture, leading the prior art to employ excess hydrogen and to obtain not pure methane but a mixture of methane and hydrogen. The abovementioned U.S. Patent 3,511,624, likewise refers to said Boudouard reaction:

$$(3) \qquad 2CO \rightarrow CO_2+C$$

and discloses the known use of steam to react

with a portion of the CO in the gas stream per reaction (2) above to assure the presence of sufficient $CO_2$ to prevent the Boudouard reaction from moving to the right and causing carbon deposits and catalyst blockages. The amount of steam thus employed is said to be at least sufficient to prevent carbon deposition, the process including the removal of the resulting carbon dioxide and any remaining steam from the mixture. While the problem of undesired carbon deposition is thus avoided in the art, the necessary adjustments to achieve this result create a further incremental limitation on the processing economy and flexibility of the prior art techniques for methane production.

In a different field, U.S. Patent No. 2544574 discloses a method for regenerating an iron catalyst which has been used for synthesizing hydrocarbons from mixtures of carbon monoxide, carbon dioxide and hydrogen. A mixture of hydrogen and steam is passed over the used catalyst at a temperature of 480 to 760°C (900 to 1400°F) for 2 to 20 hours to remove carbides, coke and wax which have been deposited on the catalyst during the hydrocarbon synthesis. During the regeneration reaction, some of the deposited carbon is converted to methane.

The present invention utilizes the catalytic disproportionation of carbon monoxide to carbon and carbon dioxide to deposit a surface layer of active surface carbon on the surface of a catalyst. A gas stream containing carbon monoxide is passed over a disproportionation catalyst at 100 to 400°C for a sufficient time to deposit the active surface layer of carbon on the catalyst without any substantial formation of inactive coke thereon. The layer of active surface carbon is thereafter contacted with steam, again at 100—400°C, to convert the active surface carbon to methane and carbon dioxide. In a less desirable embodiment, the surface layer may be contacted with hydrogen rather than with steam, to produce methane. Gas streams containing steam or hydrogen may also be used. No concentration of carbon monoxide and/or separation of inert gases is required prior to the use of dilute carbon monoxide-containing streams in the practice of the invention. At least about 12.5% and up to nearly 25% of the carbon in said carbon monoxide decomposed on the catalyst is recovered in the form of relatively pure methane. The presence of hydrogen or water in the carbon monoxide-containing feed gas stream results in methane formation upon initial contact of the feed gas with the catalyst. This methane in the gas vented from the surface layer of active surface carbon can be effectively utilized in a combustion zone for the generation of heat for steam production including steam for contacting with the active surface carbon to form methane, or other energy recovery purposes. Catalyst materials, such as nickel, cobalt, iron, ruthenium, rhenium and alloys, thereof, capable of catalyzing the disproportionation of carbon monoxide may be employed, generally in combination with catalyst support additives and/or binders to assure that the catalyst has a desired combination of activity, capacity and stability for practical, commercial operations. By carrying out the conversion of active surface carbon with high pressure steam, e.g. at 690 to 3450 k Pa (100 to 500 psi) a product stream of high pressure, relatively pure methane is produced without the necessity for employing expensive compression equipment and incurring a large consumption of energy for compressive purposes.

The present invention enables dilute carbon monoxide-containing gas streams, unacceptable for use by known processes, to be effectively and efficiently utilized for the economic production of methane. Separation of the carbon monoxide from inert gases present in such dilute carbon monoxide-containing streams is accomplished, in the practice of the present invention, without economic disadvantage vis-a-vis known processes applicable to carbon monoxide-containing gas streams not containing appreciable quantities of inert gases. The present invention is of significance, therefore, not only as a process for the production of low-cost methane but as a process uniquely capable of utilizing carbon monoxide available in gas streams not previously suitable as feed streams for the commercial production of methane.

The present invention includes the passing of a carbon monoxide-containing gas stream over a suitable catalyst under conditions such that the carbon monoxide is decomposed to form carbon dioxide and active surface carbon designated as C* and deposited as a surface layer on said catalyst according to the reaction:

$$(4) \qquad 2CO \rightarrow CO_2 + C^*$$

The surface layer of active surface carbon is thereafter converted to methane by contact with steam as follows:

$$(5) \qquad 2C^* + 2H_2O \rightarrow CH_4 + CO_2$$

The carbon efficiency of the process can be illustrated by the overall reaction (6) below that represents the total of reactions (4) and (5) as performed in the practice of the present invention:

$$(6) \qquad 4CO + 2H_2O \rightarrow 3CO_2 + CH_4.$$

Thus, 4 moles of CO are required for the production of one mole of methane in the stoichiometric relationship illustrated by reaction (6). The present invention is capable of recovering methane in amounts representing at least about 50% of the stoichiometric amount and, in preferred embodiments, at least about 80% and up to nearly 100% of said stoichiometric amount. Upon separation from the accompanying $CO_2$ by conventional means therefore, methane is recovered in the form of a low-cost, relatively pure product with the carbon values thus recovered being at least about 12.5% and up to nearly 25% of the carbon present in the carbon monoxide decomposed upon contact with the disproportionation catalyst.

Gas streams containing from about 1 to 100% by volume carbon monoxide can be utilized as the feed stream in the practice of the invention. As indicated above, the invention is uniquely capable of utilizing carbon monoxide in gas streams not suitable for known methanation techniques because of relatively high concentrations of inert gases therein. Gas streams containing carbon monoxide in amounts of from about 5% to about 50% by volume and containing at least about 5% by volume of nitrogen represent sources of carbon monoxide not previously suitable for commercial methanation operations that are highly suitable for use in the process of the invention. Those skilled in the art will readily appreciate that the gas streams should be sufficiently free from catalyst poisons to ensure adequate catalyst lifetimes. Thus, sulfur impurities should be present in very low concentrations, e.g. less than 1 ppm, preferably less than 0.2 ppm. Conventional techniques not forming a part of this invention are available in the art for removing sulfur impurities as required. If hydrogen or water vapor is also present in the feed gas stream, such gases may be converted, partially or completely, to methane by reaction with carbon monoxide under the reaction conditions. The carbon monoxide remaining after said reaction with hydrogen or water vapor will be decomposed to active surface carbon and carbon dioxide in accordance with reaction (4) above. For this reason, the hydrogen and water vapor will preferably be present in the feed gas stream in quantities less than 10% by volume of the amount of carbon monoxide present in the gas stream. It is within the scope of the invention, however, to effectively utilize any methane formed during the carbon monoxide decomposition step for heat generation purposes enhancing the overall economics of the low-cost methanation process of the invention. This aspect of the invention is discussed further herein-below.

The carbon monoxide decomposition step, in which the carbon monoxide present in the feed gas stream is decomposed to form a surface layer of active surface carbon deposited on a disproportionation catalyst, effectively serves to concentrate the carbon values to be converted to methane, regardless of the carbon monoxide content of the feed gas stream. Dilute carbon monoxide-containing gas streams can be readily employed, therefore, without the necessity for the prior concentration of the carbon monoxide as would be required in conventional techniques. The carbon dioxide formed as a result of carbon monoxide decomposition is carried away in the gas stream together with the inert gases that may be present, leaving the catalyst with a surface layer of active surface carbon values to be converted to methane. The use of a dilute carbon monoxide-containing gas stream in the process of the invention thus does not require the prior separation of the carbon monoxide content thereof from inert gases as would be required in conventional methanation techniques. This ability to utilize dilute carbon monoxide-containing gas streams constitutes a major advance in the art, permitting the production of low-cost methane from gas streams not capable of practical utilization for the economic production of methane by presently available techniques.

The decomposition of carbon monoxide over a disproportionation catalyst is generally carried out at a reaction pressure of from about 1 to about 100 atmospheres and at a reaction temperature of from about 100°C to about 400°C, preferably between about 200°C and about 300°C with space velocities of from about 1000 to about 30,000 $hr^{-1}$. Since the most useful product of the carbon monoxide decomposition, for purposes of the invention, is the solid surface layer of active surface carbons, it will usually be of no advantage to carry out the decomposition reaction at pressures much above atmospheric. The carbon monoxide-containing gas stream is passed over the catalyst for a time sufficient to deposit a surface layer of active surface carbon on the catalyst substantially without the formation of inactive coke thereon. Such inactive coke is not only itself inert under the methanation reaction conditions of the invention, but may tend to reduce the capacity of the catalyst to form active surface carbon in subsequent operations.

It should be noted that the active surface carbon formed in the practice of the invention is quite distinct from the inactive coke formed if the carbon monoxide decomposition is allowed to proceed beyond the maximum level of active surface carbon deposition. As indicated by the references to carbon deposition in the prior art description above, such inactive coke is known in the art as an undesired potential deposit on catalyst surfaces from carbonaceous feeds employed in various methanation operations. Such coke has essentially the reactivity of graphitic carbon. Its reaction with steam, for example, requires temperatures in the range of from about 600°C to about 1000°C. This reaction, which is the wellknown water gas reaction, produces CO and $H_2$ as its principal products. The active surface carbon of the present invention, on the other hand, reacts with steam at appreciably lower temperature levels to provide methane as its principal product in accordance with equation (5) above. While the prior art is concerned with the

avoidance of the undesired deposition of inactive coke on catalytic surfaces, the present invention utilizes the deposition of active surface carbon, without formation of inactive coke, to produce methane by a novel, low-cost process as described herein.

The amount of active surface carbon deposited will depend upon the surface area of the disproportionation catalyst and the operating conditions employed. Relatively low temperatures and the shortest possible residence time tend to favor the formation of the active surface carbon. Under some circumstances, particularly at higher temperatures within the operable range or with a very long residence time, the presence of CO in the gaseous effluent denotes a relatively sharp demarcation point between the deposit of the desired active surface carbon and the undesired deposition or other formation of inactive coke on the surface of the catalyst. In determining the amount of active surface carbon that can be deposited on the catalyst, therefore the point at which CO breakthrough occurs can be taken as a practical indicator of the maximum level of active surface carbon deposition. It will be understood, however, that said maximum level of deposition must be determined, for any particular embodiment, by the particular operating conditions employed, the specific catalyst utilized and the available surface area of the catalyst as applied in such embodiment.

The invention utilizes a catalyst capable of catalyzing the disproportionation of carbon monoxide. The transition metals including and to the left of nickel in the third row of the Periodic Table; including and to the left of rhodium in the fourth row thereof; and including and to the left of iridium in the fifth row thereof are capable of catalyzing said disproportionation. Preferred catalysts include nickel, cobalt, iron, ruthenium, rhenium and alloys thereof, with nickel and cobalt being most preferred on an overall technical economic basis. For purposes hereof, it will be understood that the catalyst shall include the metallic form, the oxide form, or any other suitable form of the particular catalyst employed. As the active surface carbon will be deposited in a surface layer while assuring that the decomposition reaction does not proceed to the point of inactive coke formation, a high catalyst surface area is advantageous to achieve a high surface carbon loading, enhancing the economics of the process. The catalyst employed will preferably have a surface area of at least about $10m^2/gr$, with surface areas of at least about $25m^2/gr$ being more preferred. In some embodiments, the catalyst may have an even higher surface area, ie. of at least about $50m^2/gr.$, it being appreciated that such high surface areas contribute to the attractiveness of the invention on an overall technical and economic basis. It will also be appreciated that the catalyst will generally be employed in combination with catalyst support additives and/or binding agents to assure that the catalyst has and maintains a desired combination of activity, capacity and stability for use in practical fixed or fluid bed commercial operations. It will also be understood that the surface area of the catalyst, as referred to herein, relates to the B.E.T. surface area of the catalyst composition measured after the combination of the catalyst with such additives or agents and after reduction of the catalyst to its active state.

As indicated above, inert gases present in the carbon monoxide-containing feed gas stream, together with gases formed during carbon monoxide decomposition, are vented from the reaction zone in which a surface layer of active surface carbon is deposited on the disproportionation catalyst. As a result, the carbon values in the carbon monoxide that are to be converted to methane are separated from said inert gases inherently in the practice of the invention. No prior concentration of the carbon monoxide present in dilute carbon monoxide-containing gas streams, and no separation of said carbon monoxide from inert gases such as nitrogen and argon present in said gas streams, is required. It is such requirements and the cost thereof that effectively preclude the use of dilute carbon monoxide-containing gas streams in prior art methanation techniques. The present invention achieves, in effect, such concentration essentially without a cost penalty compared to alternative processes that utilize gas streams containing a relatively high proportion of carbon monoxide therein to avoid the necessity for employing a prohibitively costly cryogenic or other separation of inerts. The present invention is particularly advantageous and achieves a major advance in the art in permitting the methanation of dilute carbon monoxide-containing gas streams and a relatively high proportion of inerts e.g. the indicated gas streams containing from about 5% to about 50% by volume carbon monoxide and at least about 5% by volume nitrogen.

The active surface carbon deposited on the catalyst, following the venting of inert gases therefrom, is contacted with steam or a steam-containing gas stream to convert said active surface carbon to methane and $CO_2$ in accordance with reaction (5) above. Reaction temperatures of from 100°C to 400°C are employed, with conversion temperatures of from about 200°C to about 350°C being generally preferred. The conversion of active surface carbon by steam may be carried out at reaction pressures of from about 1 to about 100 atmospheres. By using high pressure steam for the active surface carbon conversion, the generation of high pressure product gas stream is achieved without the need for expensive compression equipment and high energy consumption, further enhancing the economic attractiveness of the process.

The conversion of active surface carbon to

methane can, less desirably, be accomplished by contacting the surface layer of said carbon with hydrogen or a hydrogen containing gas stream under the operating conditions indicated above for conversion by steam. In this embodiment, methane is formed in accordance with the reaction:

(7) $C^*+2H_2 \rightarrow CH_4$

With the hydrogen requirements of reaction (7) being supplied from CO via the water shift reaction of reaction (2), the carbon efficiency of the process can be illustrated by the overall reaction (8) below that represents the total of reactions (2), (4) and (7) as performed in the practice of the present invention:

(8) $4\ CO+2H_2O \rightarrow 3\ CO_2+CH_4$

Thus, 4 moles of CO are again required for the production of one mole of methane in the stoichiometric relationship illustrated by reaction (8). Steam, however, is preferred for the active surface carbon conversion step because of the cost associated with the generation of hydrogen. The $CO_2$ formed in said conversion by steam can readily be seperated from the methane, if desired, by well known commercially available techniques to provide a relatively pure low-cost methane product. The Benfield aqueous alkaline scrubbing process and the Shell Sulfinol and Allied Chemical Selexol solvent extraction processes are examples of commercial techniques for removing carbon dioxide and other acid gases from gas streams.

The disproportionation catalyst will typically be mixed with a catalyst support additive or with binders to assure that the catalyst has a desired combination of activity, capacity and stability. In the absence of such additives and/or binders, nickel, for example, is relatively unstable and tends to agglomerate and sinter with resultant reduction of its surface area.

It is within the scope of the invention to employ any available support additive material capable of supporting and/or dispersing the catalyst, so as to prevent agglomeration and sintering thereof, to enhance the activity and capacity of the catalyst in continuous commercial operations. Such support additives will generally be employed in varying amounts ranging from about 0.1% to about 50% by weight of additive based on the weight of catalyst composition mixture of catalyst and additive. Examples of suitable additives are zirconia, thoria, alumina, silica and mixtures thereof, although various other materials such as rare earth oxides, may be employed for the indicated catalyst support purposes. In particular embodiments of the invention, the additive is employed in an amount within the range of from about 3% to about 15% by weight based on the weight of the catalyst composition mixture. Zirconia, alumina and silica are preferred catalyst support additives with zirconia being generally most preferred.

It will be understood that various combinations of such support additive materials, with or without binding agents, may be employed to achieve desired support and/or dispersion of the disproportionation catalyst employed in particular embodiments of the process of the invention. For example, it has been found advantageous to employ a combination of zirconia and alumina support additives. Each additive may preferably be employed in amount within the range of from about 3% to about 30% by weight of the catalyst composition mixture of catalyst and additive with the combination being employed in an amount up to about 50% by weight based on the weight of said catalyst composition. As indicated above, nickel, is the generally preferred catalyst, with the surface area of the catalyst being generally at least about $10m^2/gr$ and preferably at least about $25m^2/gr$, more preferably at least about $50m^2/gr$. Binding agents, if employed, will generally be mixed with the catalyst composition in an amount within the range of from about 5% to about 40% by weight of such binding agent based on the total weight of the catalyst composition-binder mixture. Various binding agents known in the art may be employed in a conventional manner as will readily be appreciated by those skilled in the art. Boehmite alumina a hydrous aluminum oxide, is a convenient readily available binder.

While various catalyst-support additive combinations suitable for the purposes of the invention may readily be determined by those skilled in the art, it has been found particularly convenient to employ a coprecipitated mixture of catalyst and catalyst support additive. Thermally stable coprecipitated catalyst useful for methanation reactions have heretofore been known in the art as evidence, for example by U.S. Patent No. 3,988,263 that relates to combinations of alumina with catalytic materials such as nickel. The catalyst support additive, in such embodiments, constitutes generally the hydroxide or carbonate form thereof coprecipitated with the hydroxide or carbonate of the catalyst material prior to the reduction of said catalyst hydroxide or carbonate to the active catalyst state. For purposes of the present invention, the catalyst should comprise from about 50% to about 99% of the catalyst composition mixture of catalyst and additive. Nickel and cobalt are preferred catalyst, with zirconia being the preferred catalyst support additive although it will be appreciated that alumina or other suitable support additives can also be employed.

The invention is hereinafter described with reference to particular examples presented to illustrate various aspects of the methanation process of the present invention.

Example 1

45.4 gm, 40 ml bulk of a nickel-zirconia coprecipitated catalyst composition having a weight ratio of 7 parts nickel per part of zirconia was loaded in a glass tube and was reduced at 400°C in a stream of helium-hydrogen of approximately 10:1 ratio at a flow rate of 150 ml/min for 16 hours. The catalyst had a surface area of approximately $100m^2/gm$. The temperature was adjusted to about 200°C and a mixture of helium and carbon monoxide in a 10:1 ratio i.e. a dilute carbon monoxide stream, was passed through the catalyst bed at the rate of 830 ml/min for 20 minutes at one atmos pressure. The effluent was passed through solid absorbent chips to recover by-product carbon dioxide. The temperature was increased from 200°C to about 265°C during this period. The absorbed $CO_2$ was found to measure 650 ml. No CO breakthrough in the effluent was observed.

The reactor temperature was adjusted to 250°C and superheated steam in helium, at a ratio of 1:1, was passed through the bed at the rate of 360 ml/min for 13 minutes at one atmos. During the steam reaction period, the effluent was passed through the absorbent to collect additional by-product carbon dioxide. The helium carrier gas and product methane passed the absorbent zone and product methane was determined by use of vapor phase chromatographic techniques. 398 ml of additional by-product carbon dioxide were collected. The methane product was recovered in the amount of 298 ml out of a theoretical recovery of 400 ml, i.e. with an efficiency of 74%

Example 2

A nickel-zirconia catalyst composition having a 9/1 by weight nickel/zirconia ratio was heated in a 10/1 helium/hydrogen stream at 350°C for 10 hours. After cooling in a helium stream, oxygen in helium in a 1/10 ratio was passed through the catalyst at room temperature. 18.6 gm of catalyst was then loaded into a stainless steel pressure reactor and heated at 250° for 16 hours in a 10/1 helium/hydrogen mixture at a flow rate of 150 ml/min. The thus pre-stabilized and reduced catalyst was then employed in the process of the invention by passing a 10/1 helium/carbon monoxide gas stream through the catalyst at the rate of 830 ml/min for 20 minutes, with the reactor temperature commencing at 210°C. 600 ml of carbon dioxide was collected in an absorber as in Example 1. No CO breakthrough in the effluent of the carbon monoxide decomposition reaction was observed.

The reactor was then heated to 275°C and pressurized with helium to 210 psig. Superheated pure steam, without carrier gas, was introduced into the reactor at the rate of approximately 125 ml/min for a period of 11 minutes. The effluent gas was a dispersion containing product methane, by-product carbon dioxide and helium. 162 ml of additional $CO_2$ were recovered. Product methane was recovered in the amount of 226 ml, representing a 56.5% efficiency based on the theoretical recovery of 400 ml of said product.

Example 3

A catalyst composition as in Example 2, having a nickel to zirconia ratio of 9/1, was mixed with a binder in the weight ratio of 4 parts of said catalyst composition to 1 part of binder. Boehmite alumina i.e. aluminum oxo-hydroxide, was employed as the binder. Upon mixing, the catalyst composition-binding agent paste was peptized in $HNO_3$, extruded and fired in air at 300°C. The resulting catalyst composition was reduced and stabilized as in Example 2 and was loaded into a stainless steel reactor and heated to 350°C in a helium-hydrogen stream for 16 hours. With the reactor temperature adjusted to 220°C, CO was metered into a $N_2$ stream in the ratio of 1/9, ie. producing a 10% CO-containing stream. This stream was introduced into the reactor at the rate of 1.1 l/min for 6 minutes, 315 ml of by-product $CO_2$ were recovered with no CO breakthrough observed.

The reactor was pressurized at 210 psig with nitrogen, and superheated steam, as in Example 2, was introduced at the rate of 300 ml/min for 3 minutes with the reactor temperature commencing at 270°C. 655 ml of effluent gas was recovered, at which 143.5 ml was product methane. Theoretical recovery was 172.5 ml so that the methanation efficiency was 83.2%.

Example 4

A cobalt-zirconia catalyst composition was prepared by dissolving the corresponding nitrates in water and adding an equivalent amount of sodium hydroxide in water to form the corresponding hydroxide precipitates that were filtered, washed and dried. A 9/1 weight ratio cobalt-zirconia coprecipitated catalyst composition prepared in this manner was charged, in a 30 gr. amount, in a reactor tube and reduced by heating to 400°C in a helium/hydrogen gas stream for 16 hours. The temperature of the reactor was adjusted to 229°C and a helium/carbon monoxide mixture, containing 5% CO by volume, was passed over the catalyst for a period of 5 minutes. 134 ml of byproduct $CO_2$ were measured.

With the reactor temperature adjusted to 247°C at one atm, a 30% mixture of superheated steam in helium was passed through the reactor at the rate of 220 ml/min for 5 minutes. A total of 56 ml of product methane was recovered, approaching 100% recovery. An active surface carbon loading of about 0.3% per weight of cobalt catalyst was observed, as opposed to nearly four times this catalyst loading capacity in the examples above utilizing nickel as the disproportionation catalyst. For

practical commercial applications, it would be highly desirable to employ catalyst compositions having carbon loading capacities in excess of 0.8% together with desired stability to enhance the economic advantages obtainable in fluid bed or fixed bed applications of the methanation process herein disclosed and claimed. It will be appreciated that various catalyst compositions can be formulated that, because of the relatively low proportion of active catalyst and/or the lack of sufficient available catalyst surface area, are not adequate to achieve the methanation efficiencies capable of being achieved in the practice of the invention and to which the invention is reasonably limited as herein indicated. It will also be appreciated that the effective surface area of any given catalyst composition may tend to decline over the course of long term, continuous operations while, nevertheless, achieving the significant advance in the art obtainable by the process of the invention, particularly in the utilization of dilute carbon monoxide-containing gas streams.

As indicated above, methane may be formed by the conversion of hydrogen or water upon passage of a carbon monoxide-containing gas stream that contains such hydrogen or water over the disproportionation catalyst. Such methane is vented from the catalyst having said surface layer of active surface carbon deposited thereon together with the carbon dioxide formed by the decomposition of carbon monoxide and any inert gases that may be present in the gas stream. It is within the scope of the invention to subject the vented methane to combustion in a combustion zone for heat generation purposes. The heat thus generated may be used to generate the steam utilized for the conversion of active surface carbon to methane. This ability to effectively utilize the methane formed during the carbon monoxide decomposition step, which is not readily recoverable with the product methane, further enhances the overall economic attractiveness of the methanation process of the invention. Similarly, it should be noted that any oxygen contained in the carbon monoxide-containing gas stream can be utilized for heat generation purposes, in-situ in the reactor during the carbon monoxide decomposition step or, if desired, in a preliminary combustion zone prior to the passage of the gas stream into the reactor for decomposition of the carbon monoxide content thereof.

It should be noted that a qualitative indication has been observed that the direct methanation of coke, ie the approximately thermo-neutral reaction between carbon and water according to the reaction:

(9) $2C+2H_2O \rightarrow CH_4+CO_2$

can be catalyzed by the subject disproportionation catalysts. In reducing the catalyst to the active metal state on the carbon to be converted, a $CO_2$ $(CO)_8$/toluene solution can be absorbed onto dried charcoal pellets, heated to 100°C to decompose the cobalt carbonyl, and placed under vacuum at 250°C to remove the toluene. The thus-treated pellets can then be transferred under nitrogen to a suitable steel bomb, combined therein with an appropriate quantity of water and placed in said bomb in an oven at 350°C for a few hours. Such conditions will tend to generate an autogenous pressure of about 500 psi. The bomb can then be cooled and the contents thereof measured to determine the production of methane by said direct conversion of coke and steam in the presence of said catalyst.

The process of the invention, as herein disclosed and claimed, represents a highly desirable advance in the field of methanation. It permits the production of high-Btu, pipeline-standard methane from industrial by-streams of dilute carbon monoxide not suitable for methanation by conventional methods. Many million tons of dilute by-product CO are flared each year by such basic industries as pig iron production in blast furnaces gray iron casting, petroleum cracking, and the manufacture of carbon black. Furthermore, dilute CO-containing gas streams are available from coal gasification with air and from in-situ or underground coal gasification operations. The subject invention represents an attractive technical and economic alternative to CO concentration by cryogenic or absorption techniques prior to methanation. As shown above, the process enables the carbon monoxide in dilute CO-containing gas streams to be converted and/or separated from inert gases without the need for the costly pretreatment to concentrate and separate CO that has precluded the commercial production of methane from dilute CO-containing gas streams. The subject invention can thus be effectively utilized for the production of methane from such heretofore unsuitable sources of CO, such as the effluent from the underground gasification of coal with air, the effluent from blast furnace operations, the raw synthesis gas from the oxygen-blown gasification of coal and the like. The product methane is obtained as a low-cost, relatively pure product, capable of being produced at pipeline pressures, without reliance upon hydrogen as a reactant, with the subject process appearing to be competitive with large-scale SNG production by conventional coal gasification means. While the process is directed to the production of methane, it should also be noted in passing that various other products, such as ethane or other specific organic compounds may conceivably be produced in various particular embodiments of the invention.

The production of methane by means of the subject invention provides a practical means for utilizing dilute CO-containing gas streams, and thus for reducing the waste of CO and for

recovering and reusing such CO from industrial exhaust gases to produce low-cost, high purity methane as a replacement for natural gas. The invention constitutes, therefore, a significant advance of major importance in meeting the energy requirements of industrial societies throughout the world.

## Claims

1. A process for the production of methane from a carbon monoxide-containing gas stream comprising:

(a) at 100 to 400°C passing a carbon monoxide-containing gas stream over a catalyst capable of catalyzing the disproportionation of carbon monoxide, the carbon monoxide thereby being decomposed to form carbon dioxide and an active surface carbon that is deposited on the catalyst, the gas stream being passed over the catalyst for a time sufficient to deposit a surface layer of the active surface carbon on the catalyst substantially without the subsequent formation of inactive coke thereon;

(b) at 100 to 400°C contacting the layer of active surface carbon deposited on the catalyst with (a) steam or a steam-containing gas stream or (b) hydrogen at a hydrogen-containing gas stream, thereby converting the active surface carbon to (a) methane and carbon dioxide or (b) methane.

2. A process according to claim 1 in which decomposition of carbon monoxide and the conversion of active surface carbon to methane takes place at a pressure of 1 to 100 atmospheres, the methane produced being at least 50% of the stoichiometric amount, the carbon thus recovered in the form of methane being at least 12.5% of the carbon in the carbon dioxide decomposed.

3. A process according to claim 1 or 2 in which the gas stream passed over the disproportionation catalyst contains from 5 to 50% by volume carbon monoxide and at least 5% by volume nitrogen.

4. A process according to any one of claims 1 to 3 in which the carbon monoxide decomposition temperature is from 200°C to 300°C and in which the conversion of active surface carbon by steam is at a temperature from 200°C to 350°C.

5. A process according to any one of claims 1 to 4 in which the active surface carbon conversion is carried out at a pressure of from 690 to 3450 kPa (100 to 500 psi) using steam.

6. A process according to any one of claims 1 to 5 in which the carbon monoxide-containing gas stream is passed over the catalyst until CO breakthrough occurs in the gaseous effluent from the carbon monoxide decomposition reaction.

7. A process according to any one of claims 1 to 6 in which the catalyst is selected from nickel, cobalt, iron, ruthenium, rhenium and alloys thereof.

8. A process according to claim 7 in which the catalyst comprises nickel having a surface area of at least 25 $m^2$/gm.

9. A process according to any one of claims 1 to 8 in which the catalyst is mixed with a catalyst support additive in the amount of from 0.1 to 50% by weight based on the weight of the catalyst composition mixture of catalyst and additive, the catalyst having a surface area of at least 10 $m^2$/gm.

10. A process according to claim 9 in which the additive is selected from zirconia, thoria, alumina, silica and mixtures thereof.

11. A process according to claim 10 in which the additive comprises zirconia and alumina, the zirconia and alumina each being employed in an amount of from 3% to 30% by weight, with the combination being employed in an amount up to 50% by weight, based on the weight of the catalyst composition.

12. A process according to claim 10 or 11 in which the catalyst support additive comprises the hydroxide or carbonate thereof coprecipitated with the hydroxide or carbonate of the catalyst prior to the reduction of the catalyst hydroxide or carbonate to the active catalyst state.

13. A process according to any one of claims 9 to 12 in which the catalyst composition is mixed with a binding agent in an amount of from 5% to 40% by weight based on the total weight of the catalyst composition-binder mixture.

14. A process according to any one of claims 1 to 13 in which the carbon monoxide-containing gas stream contains hydrogen or water, the hydrogen or water being converted to methane upon passage of the gas stream over the catalyst, the methane being carried away from the catalyst together with the carbon dioxide and any inert gases that may be present in the gas stream.

15. A process according to claim 14 in which the methane produced while feeding the carbon monoxide-containing gas stream is subjected to combustion in a combustion zone for heat generation purposes.

16. A process according to claim 15 in which the heat is employed for steam generation purposes, the steam being utilized for the conversion of active surface carbon to methane.

17. A process according to any one of claims 1 to 16 in which the carbon monoxide-containing gas stream comprises the effluent from the underground gasification of coal with air, the effluent from blast furnace operations, or the raw synthesis gas from oxygen-blown gasification of coal.

18. A process according to any one of claims 1 to 17 including separating the methane from the carbon dioxide in the mixture thereof produced by contacting the active carbon with steam.

## Patentansprüche

1. Verfahren zur Herstellung von Methan aus einem kohlenmonoxidhaltigen Gasstrom, dadurch gekennzeichnet, daß:

(a) ein kohlenmonoxidhaltiger Gasstrom bei 100 bis 400°C über einen zum Katalysieren der Disproportionierung von Kohlenmonoxid geeigneten Katalysator geleitet wird, wodurch das Kohlenmonoxid unter Bildung von Kohlendioxid und eines oberflächenaktiven Kohlenstoffs zerlegt wird, der auf dem Katalysator abgeschieden wird, wobei der Gasstrom über den Katalysator für eine Zeitspanne geleitet wird, die ausreicht, um eine Oberflächenschicht des oberflächenaktiven Kohlenstoffs auf dem Katalysator im wesentlichen ohne nachfolgende Ausbildung von inaktivem Koks abzulagern; und

(b) die auf dem Katalysator abgeschiedene Schicht aus oberflächenaktivem Kohlenstoff bei 100 bis 400°C mit (a) Dampf oder einem dampfhaltigen Gasstrom oder (b) Wasserstoff oder einem wasserstoffhaltigen Gasstrom in Kontakt gebracht wird, wodurch der oberflächenaktive Kohlenstoff in (a) Methan und Kohlendioxid oder (b) Methan umgewandelt wird.

2. Verfahren nach Anspruch 1, dadurch gekennzeichnet, daß die Zerlegung von Kohlenmonoxid und die Umwandlung von oberflächenaktivem Kohlenstoff in Methan bei einem Druck von 1 bis 100 Atmosphären erfolgt, das erzeugte Methan mindestens 50% der stöchiometrischen Menge ausmacht und der auf diese Weise in Form von Methan zurückgewonnene Kohlenstoff mindestens 12,5% des Kohlenstoffs in dem zerlegten Kohlendioxid ausmacht.

3. Verfahren nach Anspruch 1 oder 2, dadurch gekennzeichnet, daß der über den Disproportionierungs-Katalysator geleitete Gasstrom 5 bis 50 Vol.% Kohlenmonoxid und mindestens 5 Vol.% Stickstoff enthält.

4. Verfahren nach einem der Ansprüche 1 bis 3, dadurch gekennzeichnet, daß die Kohlenmonoxid-Zerlegungstemperatur zwischen 200°C und 300°C liegt und die Umwandlung des oberflächenaktiven Kohlenstoffs mittels Dampf bei einer Temperatur von 200°C bis 350°C durchgeführt wird.

5. Verfahren nach einem der Ansprüche 1 bis 4, dadurch gekennzeichnet, daß die Umwandlung von oberflächenaktivem Kohlenstoff bei einem Druck von 690 bis 3450 kPa (100 bis 500 psi) unter Verwendung von Dampf durchgeführt wird.

6. Verfahren nach einem der Ansprüche 1 bis 5, dadurch gekennzeichnet, daß der kohlenmonoxidhaltige Gasstrom über den Katalysator geleitet wird, bis in dem von der Kohlenmonoxid-Zerlegungsreaktion abströmenden Gas ein CO-Durchbruch erfolgt.

7. Verfahren nach einem der Ansprüche 1 bis 6, dadurch gekennzeichnet, daß der Katalysator aus der Nickel, Kobalt, Eisen, Ruthenium, Rhenium und deren Legierungen umfassenden Gruppe ausgewählt wird.

8. Verfahren nach Anspruch 7, dadurch gekennzeichnet, daß der Katalysator Nickel mit einer Oberfläche von mindestens 25 $m^2/g$ aufweist.

9. Verfahren nach einem der Ansprüche 1 bis 8, dadurch gekennzeichnet, daß der Katalysator mit einem Katalysator-trägerzusatz in einer Menge von 0.1 bis 50 Gew.% bezogen auf das Gewicht des Katalysatorzusammensetzungsgemischs aus Katalysator und Zusatzstoff gemischt wird, wobei der Katalysator eine Oberfläche von mindestens 10 $m^2/g$ hat.

10. Verfahren nach Anspruch 9, dadurch gekennzeichnet, daß der Zusatzstoff aus der Zirkoniumdioxid, Thoriumdioxid, Aluminiumoxid, Siliciumdioxid und deren Gemische umfassenden Gruppe gewählt wird.

11. Verfahren nach Anspruch 10, dadurch gekennzeichnet, daß der Zusatzstoff Zirkoniumdioxid und Aluminiumoxid aufweist, das Zirkoniumdioxid und das Aluminiumoxid jeweils in einer Menge von 3 Gew.% bis 30 Gew.% verwendet werden und die Kombination in einer Menge von bis zu 50 Gew.%, bezogen auf das Gewicht der Katalysatorzusammensetzung, vorgesehen wird.

12. Verfahren nach Anspruch 10 oder 11, dadurch gekennzeichnet, daß der Katalysatorträgerzusatzstoff das Hydroxyd oder Karbonat desselben aufweist, das zusammen mit dem Hydroxyd oder Karbonat des Katalysators vor der Reduktion des Katalysatorhydroxyds oder -karbonats zu dem aktiven Katalysatorzustand abgeschieden wird.

13. Verfahren nach einem der Ansprüche 9 bis 12, dadurch gekennzeichnet, daß die Katalysatorzusammensetzung mit einem Bindemettel in einer Menge von 5 Gew.% bis 40 Gew.% bezogen auf das Gesamfgewicht des Gemischs aus Katalysatorzusammensetzung und Bindemittel gemischt wird.

14. Verfahren nach einem der Ansprüche 1 bis 13, dadurch gekennzeichnet, daß der kohlenmonoxidhaltige Gasstrom Wasserstoff oder Wasser enthält, der Wasserstoff oder das Wasser in Methan umgewandelt werden, wenn der Gasstrom über den Katalysator geführt wird, sowie das Methan von dem Katalysator zusammen mit dem Kohlendioxid und etwaigen inerten Gasen, die in dem Gasstrom vorhanden sein können, weggeführt wird.

15. Verfahren nach Anspruch 14, dadurch gekennzeichnet, daß das während der Einspeisung des kohlenmonoxidhaltigen Gasstroms erzeugte Methan einer Verbrennung in einer Verbrennungszone zwecks Wärmeerzeugung unterzogen wird.

16. Verfahren nach Anspruch 15, dadurch

gekennzeichnet, daß die Wärme zur Dampferzeugung verwendet wird, wobei der Dampf zum Umwandeln des oberflächenaktiven Kohlenstoffs in Methan genutzt wird.

17. Verfahren nach einem der Ansprüche 1 bis 16, dadurch gekennzeichnet, daß als kohlenmonoxidhaltiger Gasstrom der von der Untertage-Vergasung von Kohle mit Luft erhaltene Gasstrom, der von Hochofenvorgängen abgehends Gasstrom oder das Rohsynthesegas aus der Kohlevergasung durch Blasen mit Sauerstoff verwendet wird.

18. Verfahren nach einem der Ansprüche 1 bis 17, dadurch gekennzeichnet, daß das Methan von dem Kohlendioxid in dem Gemisch abgetrennt wird, das durch Inkontaktbringen des aktiven Kohlenstoffs mit Dampf erzeugt wird.

**Revendications**

1. Procédé de production de méthane à partir d'un courant gazeux contenant de l'oxyde de carbone, comprenant:

(a) le passage à 100—400°C d'un courant de gaz contenant de l'oxyde de carbon sur un catalyseur capable de catalyser la dismutation de l'oxyde de carbone, ce dernier étant ainsi décomposé en formant de l'anhydride carbonique et un carbone à surface active qui est déposé sur le catalyseur, le passage du courant gazeux sur le catalyseur ayant une durée suffisante pour déposer une couche superficielle de carbone à surface active sur le catalyseur avec l'absence quasi totale de formation subséquente de coke inactif sur ce dernier;

(b) le contact à 100—400°C de la couche de carbone à surface active déposée sur le catalyseur avec (a) de la vapeur d'eau ou un courant de gaz contenant de la vapeur d'eau ou (b) de l'hydrogéne ou un courant de gaz contenant de l'hydrogéne, de manière à convertir le carbone à surface active (a) en méthane et en anhydride carbonique ou (b) en méthane.

2. Procédé suivant la revendication 1, dans lequel la décomposition de l'oxyde de carbone et la transformation du carbone à surface active en méthane ont lieu à une pression de 1 à 100 atmosphères, le méthane produit représentant au moins 50% de la quantité stoechiométrique, le carbone ainsi récupéré sous la forme de méthane représentant au moins 12,5% du carbone de l'anhydride carbonique décomposé.

3. Procédé suivant la revendication 1 ou 2, dans lequel le courant de gaz envoyé sur le catalyseur de dismutation contient 5 à 50% en volume d'oxyde de carbone et au moine 5% en volume d'azote.

4. Procédé suivant l'une quelconque des revendications 1 à 3, dans lequel la température de décomposition de l'oxyde de carbone va de 200 à 300°C et la transformation due carbone à surface active par la vapeur d'eau est effectuée à une température de 200 à 350°C.

5. Procédé suivant l'une quelconque des revendications 1 à 4, dans lequel la transformation due carbone à surface active est effectuée à une pression de 690 à 3450 kPa (100 à 500 lb/in$^2$) au moyen de vapeur d'eau.

6. Procédé suivant l'une quelconque des revendications 1 à 5, dans lequel le courant gazeux contenant de l'oxyde de carbone est envoyé sur le catalyseur jusqu'à ce que la percée de CO ait lieu dans l'effluent gazeux venant de la réaction de décomposition de l'oxyde de carbone.

7. Procédé suivant l'une quelconque des revendications 1 à 6, dans lequel le catalyseur est choisi entre le nickel, le cobalt, le fer, le ruthénium, le rhénium et leurs alliages.

8. Procédé suivant la revendication 7, dans lequel le catalyseur comprend du nickel ayant une surface spécifique d'au moins 25 m$^2$/g.

9. Procédé suivant l'une quelconque des revendications 1 à 8, dans lequel le catalyseur est mélangé avec un additif de support du catalyseur en quantité de 0.1 à 50% en poids sur la base du poids du mélange de catalyseur et d'additif représentant la composition de catalyseur, ce dernier ayant une surface spécifique d'au moins 10 m$^2$/g.

10. Procédé suivant la revendication 9, dans lequel l'additif est choisi entre la zircone, l'oxyde de thorium, l'alumine, le silicium et leurs mélanges.

11. Procédé suivant la revendication 10, dans lequel l'additif comprend de la zircone et de l'alumine, la zircone et l'alumine étant utilisées chacune en une quantité de 3 à 30% en poids, leur association étant utilisée en une quantité allant jusqu'à 50% en poids sur la base due poids de la composition de catalyseur.

12. Procédé suivant la revendication 10 ou 11, dans lequel l'additif de support du catalyseur comprend son hydroxyde ou carbonate coprécipité avec l'hydroxyde ou le carbonate du catalyseur avant la réduction de l'hydroxyde ou du carbonate du catalyseur en l'état de catalyseur actif.

13. Procédé suivant l'une quelconque des revendications 9 à 12, dans lequel la composition de catalyseur est mélangée avec un liant en une quantité de 5 à 40% en poids sur la base du poids total du mélange formé de la composition de catalyseur et du liant.

14. Procédé suivant l'une quelconque des revendications 1 à 13, dans lequel le courant de gas contenant de l'oxyde de carbone renferme de l'hydrogène ou de l'eau, l'hydrogène ou l'eau étant transformé en méthane lors du passage du courant de gaz sur le catalyseur, le méthane étant balayé du catalyseur en même temps que l'anhydride carbonique et que tous gaz inertes que peuvent être présents dans le courant gazeux.

15. Procédé suivant la revendication 14, dans lequel le méthane produit pendant que le courant de gaz contenant de l'oxyde de carbons est chargé, est soumis à une combustion dans

une zone de combustion en vue d'engendrer de la chaleur.

16. Procédé suivant la revendication 15, dans lequel la chaleur est utilisée pour produire de la vapeur, cette vapeur étant utilisée pour convertir du carbone à surface active en méthane.

17. Procédé suivant l'une quelconque des revendications 1 à 16, dans lequel le courant de gaz contenant de l'oxyde de carbone est l'effluent de la gazéification souterraine de charbon par l'air, l'effluent de la conduite de hauts fourneaux ou le gaz brut de synthèse de la gazéification du charbon sous courant d'oxygène.

18. Procédé suivant l'une quelconque des revendications 1 à 17, comprenant la séparation du méthane de l'anhydride carbonique dans son mélange produit par contact du carbone actif avec de la vapeur d'eau.